# EUROPEAN PATENT APPLICATION

(11) **EP 0 622 625 A2**
(43) Date of publication of application: **02.11.1994**
(21) Application number: 94106505.4
(22) Date of filing: 26.04.1994
(51) Int. Cl.: G01N 21/35, G01N 21/53

(54) **System for monitoring air quality**

(30) Priority: 27.04.1993 US 52984
(71) Applicant: Hughes Aircraft Company, Los Angeles, California 90080-0028 (US)
(72) Inventor: Kert, John, Santa Monica, CA 90402 (US); Sorbo, Nelson W., Canoga Park, CA 91304 (US); Sapre, Alex, Fullerton, CA 92635 (US)
(74) Representative: KUHNEN, WACKER & PARTNER

(57) **Abstract**

Air quality in a geographic area is monitored through an integrated network (10) of a central site (80) for receiving telemetrically-transmitted air-content data and multiple air-content sensors (20, 30, 40, 50, 60, 70) distributed throughout the geographic area. The air-content sensors (20, 30, 40, 50, 60, 70) telemetrically-transmit air-content data to the central information receiving site (80). The distributed sensors (20, 30, 40, 50, 60, 70) not only monitor air in general but are also located at pollutant emission sites such as industrial exhaust flues (61), vehicle thoroughfares (51) which are generally traveled by exhaust-emitting vehicles, and sites through which gaseous substances regularly flow and are likely to escape. The location which is the source of telemetrically-transmitted air-content data may be identified by encoding the signal (22, 32, 42, 52, 62, 72) transmitted from the source. Telemetric-transmission may be accomplished by satellite (13) link, cellular relay, or radio-transmission.

## Description

### TECHNICAL FIELD OF THE INVENTION

The present invention relates to a system for monitoring air quality, and more particularly to an integrated system for real-time assessment of air quality.

### BACKGROUND OF THE INVENTION

The quality of air greatly affects the sustenance of plant and animal life in a geographic area. In particular, air quality affects the ability of humans to comfortably live or survive in a geographic area. Air quality is affected by the types and concentrations of gaseous substances that are present in the normal unadulterated mixture of gases that make up air. The quality of air at a given instant is a function of pollutants present in air and pollutants entering air. It is important to monitor air quality to detect any changes for several reasons. One reason is that a change in air quality may result in a condition that is less than optimum for human habitation of the area. In this instance, individuals may experience severe health problems in attempting to breathe degraded air or may not be able to live if air in the area is breathed over a period of time. It is desirable in these instances to issue health warnings or alerts. Another reason for monitoring air quality is that a change in air quality due to pollutants may be an indication of an impending or occurring environmental or man-made disaster. For example, gaseous emissions from a volcano may signal an impending or occurring eruption. Similarly, an accident at a nuclear plant or in a gas pipeline will cause characteristic gases to enter the air.

A problem in monitoring air quality is that it is difficult to accurately assess air quality in a geographic area where the content of the air frequently changes due to pollutants periodically or regularly entering the air. Likely emitters of gaseous pollutants are vehicle exhausts, industrial flues, and mechanical members such as flanges and valves of pipelines which restrain or direct the flow of gaseous substances.

### SUMMARY OF THE INVENTION

It is an object of the invention to provide a means for analyzing air-quality on a continuous, real-time basis.

It is a further object of the invention to provide a means for real-time analysis of air quality by integrating air-quality information from multiple monitoring sites including pollutant emission sites.

According to a preferred embodiment of the present invention, air quality in a geographic area is monitored through an integrated network of a central site for receiving telemetrically-transmitted air-content data and multiple air-content sensors distributed throughout the geographic area. The air-content sensors telemetrically-transmit air-content data to the central information receiving site. The distributed sensors not only monitor air in general but are also located at pollutant emission sites such as industrial exhaust flues, vehicle thoroughfares which are generally traveled by exhaust-emitting vehicles, and sites through which gaseous substances regularly flow and are likely to escape. The location which is the source of telemetrically-transmitted air-content data may be identified by encoding the signal transmitted from the source. Telemetric-transmission may be accomplished by satellite link, cellular relay, or radio-transmission.

Other aspects, objects, features, and advantages of the present invention will become apparent to those skilled in the art upon reading the detailed description of preferred embodiments.

### BRIEF DESCRIPTION OF THE DRAWINGS

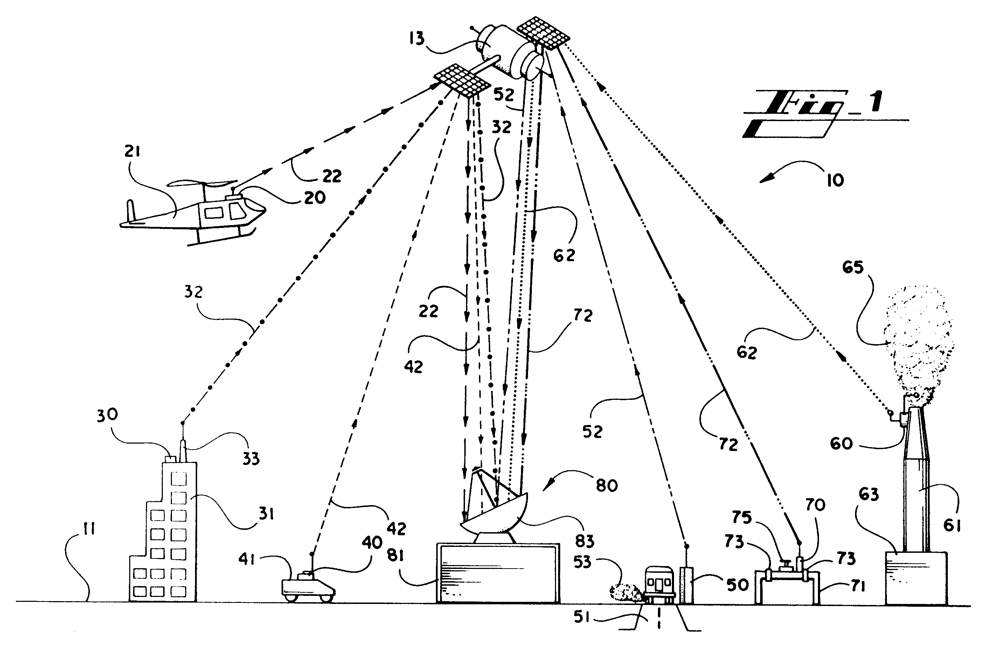

Fig. 1 is a graphic illustration of a system for monitoring air quality according to a preferred embodiment of the invention.

### DETAILED DESCRIPTION OF PREFERRED EMBODIMENTS OF THE INVENTION

While the specification concludes with claims particularly pointing out and distinctly claiming the subject matter which is regarded as the present invention, the invention will now be described with reference to the following description of embodiments taken in conjunction with the accompanying drawings.

The invention integrates multiple air-monitoring capabilities into a single system which provides real-time analysis of air-content. Referring to Fig. 1, therein is illustrated in graphic form a system for monitoring air quality 10 in accordance with a preferred embodiment of the invention. Fig. 1 graphically depicts a geographic area. The air-content at several sites is analyzed and integrated into the system 10. The content of air above the ground 11 is analyzed by an airborne sensor 20. The purpose of the airborne sensor 20 is to analyze air at different altitudes above the ground 11 and at different locations in the geographic region. A convenient means of performing airborne analysis is by mounting the airborne sensor 20 upon an air vehicle such as a helicopter 21. The helicopter 21 provides the advantage of being able to hover to obtain a reading and analysis at a fixed mid-air location. The airborne sensor 20 is able to take readings at a variety of mid-air locations in a short period of time.

Each sensor, such as the airborne sensor 20, used in the system 10 is a commonly-used air-content analyzer such as an Infrared Laser Absorption Based System (also known by the acronym LIDAR) or either of the so-called Fourier Transform (FTIR) or Non-Dispersive Infrared (NDIR) systems. However, each sensor in the system 10 instanteously telemetrically transmits its air-content data to a central receiving source. Telemetric-transmission includes signal transmission through a satellite link, through cellular relay stations (as are commonly used in mobile telephones), and simple radio transmission as commonly used in police cars. In the system 10 illustrated in the preferred embodiment of Fig. 1, a satellite 13 link is employed to transmit the air-content data signal 22 from the airborne sensor 20 to an air-quality management center 80. In the graphic illustration of the preferred embodiment shown, the signal 22 from the airborne sensor 20, as are other signals, is collected by a parabolic dish antenna 83 atop the receiving station 81 of the air-quality management center 80.

A relatively fixed ground-base air-content sensor 30 may be placed atop a structure such as a building 31. A convenient means of transmitting the air-content data signal 32 from the ground base sensor 30 is through an antenna tower 33. Antenna towers 33 are commonly found atop tall buildings and may serve as a convenient means for transmittal of the signal. The air-content data signal 32 from the ground-based sensor 30 is also telemetrically-transmitted to the air quality management center via the satellite 13 link.

Air-content from various sites at ground 11 level may be analyzed through the use of a ground-vehicle-based air-content sensor 40. Such a ground-vehicle sensor 40 may conveniently be mounted upon a vehicle such as a van 41 or car. Once again, the air-content data signal 42 from the ground-vehicle-based sensor 40 is telemetrically-transmitted via satellite 13 link to the dish antenna 83 at the receiving station 81.

In order to accurately assess air quality in a geographic area, not only must the air content in general be analyzed but also the nature and amounts of pollutants entering the air must be assessed. The airborne 20, ground-based 30, and ground-vehicle-based 40 sensors analyze and transmit data regarding air-content at precise points at precise instants, however, in the next instant the air-content at a particular point may change due to pollutants which have previously entered the air but whose effect has not yet been monitored. In the system 10 illustrated in Fig. 1, sensors 50, 60, and 70 are strategically located at potential pollutant emission sites. A roadside vehicle-emission sensor 50 placed next to a highway 51 analyzes the content of air at a point along the highway 51. A mixture of gases contained in air sampled from this location will be heavily laced with gases emitted from vehicle exhausts. As these polluting gases enter the atmosphere in a particular geographic area, the quality of air degrades as these pollutants mix with the air. As before, the air-content data signal 52 is transmitted via satellite 13 link to the dish antenna 83 at the air quality management center 80.

The system 10 monitors the exhaust flue of industrial sites as another pollutant source. In the system 10 illustrated in Fig. 1, an air-content sensor 60 monitors the smoke stack 61 of a factory 63 to assess the types and rates of emission of pollutants. The air-content data signal 62 is telemetrically-transmitted via the satellite 13 link to the air quality control center 80. A so-called fugitive-emission sensor 17 is placed at the site of a normally-closed system such as a gas pipeline 71 to monitor escaping pollutants. The sensor 70 is shown in proximity to the most likely points of pollutant leakage, namely, flanges 73 and valves 75. Any leaking pollutant is detected by the sensor 70 and resulting air-content data 72 is telemetrically-transmitted via the satellite 13 link to the dish antenna 83 at the receiving station 81.

In order to provide an accurate real-time analysis of the air-content in a geographic area as illustrated, the air quality management center 80 must be able to distinguish the sources of the various signals 22, 32, 42, 52, 62, 72 received. Identification of each signal source is achieved by encoding each data signal. A convenient method for encoding each signal is to transmit each signal in digital form with a binary code indicative of each source embedded in the signal. When each signal 22, 32, 42, 52, 62, 72 is bit-encoded in this manner, the source of each can be readily ascertained, as graphically illustrated in Fig. 1.

The system 10 is able to present a real-time analysis of air-content in a geographic area by integrating the signal responses 22, 32, 42, 52, 62, 72 received from respective multiple air-content sensors 20, 30, 40, 50, 60, 70. The system 10 is capable of determining air quality at a given instant, changing air quality over a period, and predictable future air quality based upon real-time empirical data.

As should be apparent from the foregoing specification, the invention is susceptible of being modified with various alterations and modifications which may differ from those which have been described in the preceding specification and description. Accordingly, the following claims are intended to cover all alterations and modifications which do not depart from the spirit and scope of the invention.

## Claims

1. A system for monitoring air quality in a geographic area comprising:
means for receiving telemetrically-transmitted air-content data;
at least one ground-based air-content sensing means in the geographic area, having means for telemetrically-transmitting air-content data to said means for receiving telemetrically-transmitted air-content data;
at least one air-content sensing means proximate a source in the geographic area from which gases to be detected may escape, having means for telemetrically transmitting air-content data to said means for receiving telemetrically-transmitted air-content data; and
at least one air-content sensing means mounted upon a vehicle traveling in the geographic area, having means for telemetrically-transmitting air-content data to said means for receiving telemetrically-transmitted air-content data.

2. A system for monitoring air quality in a geographic area comprising:
means for receiving telemetrically-transmitted air-content data;
at least one air-content sensing means mounted upon an airborne vehicle traveling in the geographic area, having means for telemetrically transmitting air-content data to said means for receiving telemetrically-transmitted air-content data;
at least one ground-based air-content sensing means in the geographic area, having means for telemetrically transmitting air-content data to said means for receiving telemetrically-transmitted air-content data;
at least one air-content sensing means mounted upon a ground vehicle traveling in the geographic area, having means for telemetrically transmitting air-content data to said means for receiving telemetrically-transmitted air-content data;
at least one air-content sensing means positioned proximate a thoroughfare in the geographic area, having means for telemetrically transmitting air-content data to said means for receiving telemetrically-transmitted air-content data;
at least one air-content sensing means mounted proximate an exhaust of an industrial flue in the geographic area, having means for telemetrically transmitting air-content data to said means for receiving telemetrically-transmitted air-content data; and
at least one air-content sensing means positioned proximate a mechanical member from which gases may escape in the geographic areas, having means for telemetrically transmitting air-content data to said means for receiving telemetrically-transmitted air-content data.

3. The invention of claim 2, each said means for telemetrically transmitting air-content data to said means for receiving telemetrically-transmitted air-content data of said respective air-content sensing means mounted upon an air vehicle, ground-based air-content sensing means, air-content sensing means mounted upon a ground vehicle, air-content sensing means positioned proximate a thoroughfare, air-content sensing means mounted proximate an exhaust of an industrial flue, and air-content sensing means positioned proximate a mechanical member from which gases may escape, comprising means for telemetrically transmitting bit-coded air-content data to said means for receiving telemetrically-transmitted air-content data so that a source of each bit-coded transmission is identifiable by binary bit-coding distinct to said source.

4. The invention of claim 2, said means for receiving telemetrically-transmitted air-content data comprising means for receiving telemetrically-transmitted air-content data from a satellite and said means for telemetrically transmitting air-content data to said means for receiving telemetrically-transmitted air-content data of said respective air-content sensing means mounted upon an air vehicle, ground-based air-content sensing means, air-content sensing means mounted upon a ground vehicle, air-content sensing means positioned proximate a thoroughfare, air-content sensing means mounted proximate an exhaust of an industrial flue, and air-content sensing means positioned proximate a mechanical member from which gases may escape, comprising means for telemetrically transmitting air-content data to the satellite.

5. The invention of claim 2, said central data receiving means for receiving telemetrically transmitted air-content data comprising means for receiving direct radio-transmitted air-content data and said means for telemetrically transmitting air-content data to said means for receiving telemetrically-transmitted air-content data of said respective air-content sensing means mounted upon an air vehicle, ground-based air-content sensing means, air-content sensing means mounted upon a ground vehicle, air-content sensing means positioned proximate a thoroughfare, air-content sensing means mounted proximate an exhaust of an industrial flue, and air-content sensing means positioned proximate a mechanical member from which gases may escape, comprising means for telemetrically transmitting air-content data by direct radio wire transmission to said means for receiving radio-transmitted air-content date.

6. The invention of claim 2, said means for receiving telemetrically transmitted air-content data comprising means for receiving telemetrically-transmitted air-content data from at least one cellular relay station and said means for telemetrically transmitting air-content data to said means for receiving telemetrically-transmitted air-content data of said respective air-content sensing means mounted upon an air vehicle, ground-based air-content sensing means, air-content sensing means mounted upon a ground vehicle, air-content sensing means positioned proximate a thoroughfare, air-content sensing means mounted proximate an exhaust of an industrial flue, and air-content sensing means positioned proximate a mechanical member from which gases may escape, comprising means for telemetrically transmitting air-content data to at least one cellular relay station for retransmission to said means for receiving telemetrically-transmitted air-content data from at least one cellular relay station.

7. A system for monitoring air quality in a geographic area comprising:
means for receiving telemetrically-transmitted binary-coded air-content data;
at least one air-content sensing means mounted upon an airborne vehicle traveling in the geographic area, having means for telemetrically transmitting binary-coded air-content data to said means for receiving telemetrically-transmitted binary-coded air-content data;
at least one ground-based air-content sensing means in the geographic area, having means for telemetrically transmitting binary-coded air-content data to said means for receiving telemetrically-transmitted binary-coded air-content data;
at least one air-content sensing means mounted upon a ground vehicle traveling in the geographic area, having means for telemetrically transmitting binary-coded air-content data to said means for receiving telemetrically-transmitted binary-coded air-content data;
at least one air-content sensing means positioned proximate a thoroughfare in the geographic area, having means for telemetrically transmitting binary-coded air-content data to said means for receiving telemetrically-transmitted binary-coded air-content data;
at least one air-content sensing means mounted proximate an exhaust of an industrial flue in the geographic area, having means for telemetrically transmitting binary-coded air-content data to said means for receiving telemetrically-transmitted air-content data; and
at least one air-content sensing means positioned proximate a mechanical member from which gases may escape in the geographic areas, having means for telemetrically transmitting binary-coded air-content data to said means for receiving telemetrically-transmitted binary-coded air-content data.

8. The invention of claim 7, said means for receiving telemetrically-transmitted binary-coded air-content data comprising means for receiving telemetrically-transmitted binary-coded air-content data from a satellite and said means for telemetrically transmitting binary-coded air-content data to said means for receiving telemetrically-transmitted binary-coded air-content data of said respective air-content sensing means mounted upon an air vehicle, ground-based air-content sensing means, air-content sensing means mounted upon a ground vehicle, air-content sensing means positioned proximate a thoroughfare, air-content sensing means mounted proximate an exhaust of an industrial flue, and air-content sensing means positioned proximate a mechanical member from which gases may escape, comprising means for telemetrically transmitting binary-coded air-content data to the satellite.

9. The invention of claim 7, said central data receiving means for receiving telemetrically-transmitted binary-coded air-content data comprising means for receiving direct radio-transmitted binary-coded air-content data and said means for telemetrically transmitting binary-coded air-content data to said means for receiving telemetrically-transmitted binary-coded air-content data of said respective air-content sensing means mounted upon an air vehicle, ground-based air-content sensing means, air-content sensing means mounted upon a ground vehicle, air-content sensing means positioned proximate a thoroughfare, air-content sensing means mounted proximate an exhaust of an industrial flue, and air-content sensing means positioned proximate a mechanical member from which gases may escape, comprising means for telemetrically transmitting binary-coded air-content data by direct radio transmission to said means for receiving direct radio-transmitted binary-coded air-content date.

10. The invention of claim 7, said means for receiving telemetrically transmitted binary-coded air-content data comprising means for receiving telemetrically-transmitted binary-coded air-content data from at least one cellular relay station and said means for telemetrically transmitting binary-coded air-content data to said means for receiving telemetrically-transmitted binary-coded air-content data of said respective air-content sensing means mounted upon an air vehicle, ground-based air-content sensing means, air-content sensing means mounted upon a ground vehicle, air-content sensing means positioned proximate a thoroughfare, air-content sensing means mounted proximate an exhaust of an industrial flue, and air-content sensing means positioned proximate a mechanical member from which gases may escape, comprising means for telemetrically transmitting binary-coded air-content data to at least one cellular relay station for retransmission to said means for receiving telemetrically-transmitted binary-coded air-content data from at least one cellular relay station.
